# EUROPEAN PATENT APPLICATION

(11) **EP 2 924 019 A1**
(43) Date of publication of application: **30.09.2015**
(21) Application number: 14161465.1
(22) Date of filing: 25.03.2014
(51) Int. Cl.: C07C 29/149, C07C 67/475

(54) **PROCESS FOR THE MANUFACTURE OF A SATURATED ALCOHOL**

(71) Applicant: DSM IP Assets B.V., 6411 TE Heerlen (NL)
(72) Inventor: Lefort, Laurent, 6100 AA ECHT (NL); Cosimi, Elena, 6100 AA ECHT (NL); Schmitkamp, Mike, 6100 AA ECHT (NL)
(74) Representative: Pacifico, Jessica

(57) **Abstract**

The present invention relates to a process for the manufacture of a saturated primary alcohol from an unsaturated aliphatic ester comprising the steps of:
a) Providing an aliphatic ester having at least one carbon-carbon double bond;
b) Carrying out a metathesis of said ester in the presence of a ruthenium carbenebased catalyst thereby obtaining a first reaction mixture;
c) Adding a ligand and a base to the first reaction mixture, wherein the ligand comprises at least one donor atom chosen from the group consisting of a nitrogen atom and a phosphorus atom thereby obtaining a second reaction mixture comprising an ester product resulting from the metathesis reaction ;
d) Carrying out a homogeneous hydrogenation of the ester-product resulting from the metathesis, thereby obtaining a saturated primary alcohol.

Further, the present invention relates to a catalyst for the hydrogenation of esters and to a process for the hydrogenation of esters using said catalyst.

## Description

The present invention relates to a process for the manufacture of a saturated primary alcohol from an aliphatic ester having at least one carbon-carbon double bond. Further, the present invention relates to a catalyst for the hydrogenation of esters and to a process for the hydrogenation of esters using said catalyst.

When considering the preparation of α,ω-diols, the metathesis reaction of unsaturated esters followed by its hydrogenation is known as recited by Multu et al. RSC Adv., 2013, 3, pp.4927-4934. In this process, one catalyst is used in the metathesis step leading to a new unsaturated diester and a second catalyst is used in the hydrogenation step leading to the hydrogenation of the ester functional groups into alcohol functional groups. Such a process presents some drawbacks when mconsidering the implementation of the process in industrial plants. Namely, the process must occur in two steps, involving the removal of the first catalyst used in the metathesis reaction step, before carrying out the hydrogenation in the second step. Further, the hydrogenation, or reduction, of esters is rather difficult compared to ketones and aldehydes, esters are relatively resistant to reduction. With catalytic hydrogenation, esters can be hydrogenated to primary alcohols according to the reaction RCO₂R' + 2 H₂ → RCH₂OH + R'OH. Typical catalysts for the hydrogenation of esters into alcohols are heterogeneous copper chromite modified with various additives (zinc, iron, barium...), which presents a non-negligible toxicity. Prior to the development of catalytic hydrogenation, esters were reduced on a large scale using the Bouveault-Blanc reduction, using sodium in the presence of proton sources. Lithium aluminium hydride is also used to reduce esters to two primary alcohols. However, the high reactivity of metallic sodium and lithium aluminium hydride prevents their use in today's large industrial plants. Compared to lithium aluminum hydride, sodium borohydride may be a possible alternative, however it slows down the reduction of esters into alcohols as compared to other catalysts such as lithium aluminum hydride. Additionally, there are non-negligible technical differences when considering heterogeneous (catalyst in a different phase than the reactants) or homogeneous (catalyst in the same phase as the reactants) catalysis for the hydrogenation of an ester. The hydrogenation of an ester into an alcohol with a heterogeneous catalyst involves drastic reaction conditions (minimum pressures of 100-200 bar and temperatures of at least 250°C), while the reaction conditions in homogeneous catalysis are normally milder, but directed to activated esters. Further, the conversion rate (or yield) may however be much lower in homogeneous catalysis (as little as 5 to 10%) than in heterogeneous catalysis (70 to 90%).

One goal of the present invention, among other goals, is to provide a process for the manufacture of a saturated primary alcohol from an aliphatic ester having at least one carbon-carbon double bond which is suitable for industrial manufacturing processes and which does not present the drawbacks of the prior art, specifically providing a process in which the ester is first transformed via a metathesis reaction using a catalyst that can be maintained in the reaction mixture and used further in the hydrogenation step. Another goal of the present invention is to provide a process wherein no toxic (for the environment, and/or for human beings and animals) catalyst is used, having a high conversion rate and an advantageously fast reaction time.

The above-mentioned goals are achieved by the process according to the present invention, for the manufacture of a saturated primary alcohol from an unsaturated aliphatic ester comprising the steps of:
a) Providing an aliphatic ester having at least one carbon-carbon double bond;
b) Carrying out a metathesis of said ester in the presence of a ruthenium carbene-based catalyst thereby obtaining a first reaction mixture;
c) Adding a ligand and a base to the first reaction mixture obtained after step b), wherein the ligand comprises at least one donor atom chosen from the group consisting of a nitrogen atom and a phosphorus atom thereby obtaining a second reaction mixture comprising an ester product resulting from the metathesis reaction ;
d) Carrying out a homogeneous hydrogenation of the ester-product resulting from the metathesis, thereby obtaining a saturated primary alcohol.

Accordingly, the amount of catalyst in the process according to the present invention is equal to, or even lower to the known industrial manufacture processes, thereby providing practical and technical advantages such as, lighter loading of catalyst into a reactor, the removal of less catalyst at completion of the reaction. One further advantage of the process according to the present invention is that the catalyst used in step b) remains in the reactive mixture obtained after step b) (designated as the first reaction mixture). In other words, the catalyst used in the metathesis step is further used (after chemical transformation in step c)), in the homogeneous hydrogenation in step d). This first aspect of the present invention provides a process for the preparation of primary alcohols (such as alkanols or alkanediols): the metathesis of a mono-functionalized ester followed by ester hydrogenation in which the ester hydrogenation catalyst is formed by in-situ modification of the metathesis catalyst. The process is particularly fitted for the production of long chain aliphatic alcohols, such as alcohols having an -OH function is located at an extremity of a hydrocarbon chain, i.e. primary alcohols (for example α-alcohols or α,ω-diols) from renewables fatty acid esters. In the process according to the present invention, the total conversion yield of the two reactions (metathesis and hydrogenation) according to the present invention is particularly high, i.e. above 50%: in particular in the range from 55% to 95% relative to the molar amount of ester provided in step a). The conversion yield of the metathesis step is at least 65% and can reach conversion yield as high as 90%. The yield of the hydrogenation step according to the present invention is at least 85 % and can reach conversion yield as high as 99%. One additional advantage of the process according to the present invention is that all chemical groups which may be sensitive to hydrogenation can be hydrogenated (or reduced) during the hydrogenation step. If desired, the process according to the present invention can also be tuned to maintain some unsaturation on the alcohol obtained by the process.

The process according to the present invention may also be carried out with other starting compounds than aliphatic unsaturated esters, such as esters which are alkenyl-substituted aromatic esters, or between an aliphatic unsaturated ester and an alkyne, or an aliphatic unsaturated aldehyde, or an alkenyl-substituted aromatic aldehyde.

Olefin metathesis is an organic reaction that entails the redistribution of fragments of alkenes (olefins) by the scission and regeneration of carbon-carbon double bonds. The groups bound to the carbon atoms of the double bond are exchanged between molecules, to produce two new molecules containing double bonds with swapped groups. Whether a cis isomer or trans isomer is formed in this type of reaction is determined by the orientation of the molecules when they coordinate to the catalyst, as well as by the substituents on the double bond of the newly forming molecule. A self-metathesis is the intermolecular reaction between two identical molecules comprising a carbon-carbon double bond. A cross-metathesis is the intermolecular reaction between two different molecules comprising a carbon-carbon double bond. A ring closing metathesis is a intramolecular reaction between two C-C double bonds inside the same molecule. Accordingly, in step b), the product obtained from the metathesis reaction is an (different) ester (comprising at least one carbon-carbon double bond) than the ester provided in step a) and is therefore designated as "the ester product resulting from the metathesis reaction". Since the ester product resulting from the metathesis reaction after step b) is in the presence of other components, such as the catalyst and eventually a solvent, it can be designated as "first reaction mixture", the mixture obtained after step b). When carrying out step c), the ester product of the metathesis is not hydrogenated, yet (not reacted yet) in step c). Step c) involved the addition of a ligand and a base, thereby forming a second reaction mixture. The second reaction mixture thus still contains the ester product resulting from the metathesis reaction and is submitted to the hydrogenation in step d). The product of the hydrogenation is the hydrogenated ester product of the metathesis reaction: a primary alcohol having saturated carbon-carbon bonds. The process of the present invention is not suitable for providing secondary or tertiary alcohols.

In the context of the present invention, a saturated alcohol is manufactured by the process according to the present invention, starting from an aliphatic unsaturated ester (starting compound). The term "aliphatic ester" is to be understood as a linear, branched or cyclic hydrocarbon chain which apart from the ester functionality is optionally substituted with other functional groups as defined below. The aliphatic ester comprising at least one ester function (-COO-alkyl/aryl) and having at least one carbon-carbon double bond (also designated as C=C, i.e. unsaturation) that can take part to a metathesis reaction. In the preferred case where the aliphatic ester comprises only one ester function, it can be designated as a monoester. The aliphatic ester can also comprise more than one ester function, such as two, three, four, five, six ester functions or more. In the context of the present invention, the aliphatic ester processed according to the present invention is transformed into an alcohol after the hydrogenation step (step d)). In other words, the hydrogenation of all the ester functions of the aliphatic ester introduced in step a) is carried out in step d). The aliphatic ester may be any ester having between 2 and 40 carbon atoms in the whole molecule and may be substituted at any position along it chain by a halogen (such as -F, -Cl, -Br, -I), methyl, ethyl, phenyl group, an hydroxyl (-OH), or a carbonyl containing substituent (-(C=O)- containing substituent can be such as an aldehyde, an amide, an ester). The process according to the present invention provides a high conversion rate when the aliphatic ester has at least 8 carbon atoms, preferably at least 10 carbon atoms, more preferably at least 12 carbon atoms. Accordingly, in the process according to the present invention, the ester can advantageously be an ester of an unsaturated fatty acid. In the context of the present invention, the at least one carbon-carbon double bond (C=C) is to be understood as at least one C=C bond which can react in a metathesis reaction. The reaction in the metathesis reaction depends on the degree of substitution on the carbon-carbon double bond and on the electron withdrawing properties of the substituents (sterically hindered C=C and/or comprising substituents which are electron withdrawing provide less suitable carbon-carbon double bonds for metathesis reactions). Examples of suitable substituents around the C=C bond are one or more: -H, methyl, ethyl, or a linear alkyl. In the context of the present invention the term 'at least one carbon-carbon double bond' is to be understood as one or more, two or more, three or more, four or more, five or more, six or more carbon-carbon double bonds. Examples of unsaturated fatty acids can be decenoic acid CH=CH(CH₂)₇COOH, myristoleic acid CH₃(CH₂)₃CH=CH(CH₂)₇COOH, palmitoleic acid CH₃(CH₂)₅CH=CH(CH₂)₇COOH, sapienic acid CH₃(CH₂)₈CH=CH(CH₂)₄COOH, oleic acid (CH₃(CH₂)₇CH=CH(CH₂)₇COOH), elaidic acid CH₃(CH₂)₇CH=CH(CH₂)₇COOH, vaccenic acid CH₃(CH₂)₅CH=CH(CH₂)₉COOH, linoleic acid CH₃(CH₂)₄CH=CHCH₂CH=CH(CH₂)₇COOH, linoelaidic acid CH₃(CH₂)₄CH=CHCH₂CH=CH(CH₂)₇COOH (geometric isome of linoleic acid), α-linolenic acid CH₃CH₂CH=CHCH₂CH=CHCH₂CH=CH(CH₂)₇COOH, arachidonic acid CH₃(CH₂)₄CH=CHCH2CH=CHCH2CH=CHCH₂CH=CH(CH₂)₃COOH, eicosapentaenoic acid. (CH₃CH₂CH=CHCH₂CH=CHCH₂CH=CHCH₂CH=CHCH₂CH=CH(CH₂)₃COOH) erucic acid (CH₃(CH₂)₇CH=CH(CH₂)ₙCOOH), docosahexaenoic acid (CH₃CH₂CH=CHCH₂CH=CHCH₂CH=CHCH₂CH=CHCH₂CH=CHCH₂CH=CH(CH₂)₂CO OH). Preferably, the ester mentioned in the context of the present invention is a methyl, an ethyl, a propyl, i-propyl, butyl, methyl-butyl, i-butyl, t-butyl, cyclohexyl, or a phenyl ester of a carboxylic acid. In the context of the present invention, the catalyst is a ruthenium carbene-based catalyst. The carbene-based catalyst is designated as a catalyst comprising at least one double M-C (or (M=C) bond between the metal (M) and one carbon of a ligand (C). M is herewith described as ruthenium, but it can be also osmium, molybdenum, tungsten.

In the context of the present invention, the term "alkyl" designates, unless otherwise indicated, any linear or branched alkyl and comprises between 1 and 40 carbon atoms. The term "cycloalkyl" designates, unless otherwise indicated, any cyclic alkyl and comprises between 1 and 40 carbon atoms. the term "aryl" designates, unless otherwise indicated, any aromatic hydrocarbon and comprises between 1 and 40 carbon atoms. When referred to terms such as alkyl, cycloalkyl, and aryl, it is to be understood a rest, or radical, alkyl, cycloalkyl and aryl respectively. The alkyl, cycloalkyl and aryl may or may not be substituted by an alkyl, cycloalkyl and aryl, a functional group such as an organic function, a halogen atom.

According to the present invention, the catalyst can be a ruthenium-based catalyst, preferably the catalyst has the formula (I): 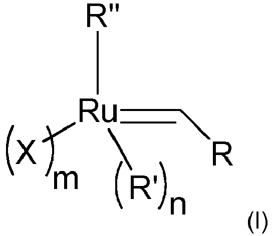 wherein
m and n are independently numerals between 0 and 2,
X is a ionic ligand chosen from the group consisting of halides, phenoxy, alkoxy and hydroxyl,
R is chosen from the group consisting of an unsubstituted linear alkyl, a substituted linear alkyl, an unsubstituted aryl and a substituted aryl,
R' is chosen from the group consisting of an unsubstituted trialkylphosphine, a substituted trialkylphosphine, an unsubstituted tricycloalkylphosphine, a substituted tricycloalkylphosphine, an unsubstituted triarylphosphine, a substituted triarylphosphine, an unsubstituted pyridine, a substituted pyridine, an unsubstituted pyrazine and a substituted pyrazine;
   and
R" is chosen from the group consisting of a substituted imidazole-2-ylidene or a substituted dihydroimidazole-2-ylidene, an unsubstituted trialkylphosphine, a substituted trialkylphosphine, an unsubstituted tricycloalkylphosphine, a substituted tricycloalkylphosphine, an unsubstituted triarylphosphine, a substituted triarylphosphine.
X, R and R" are covalently bond ligands. R may further have an additional dative bond towards the ruthenium metallic center (Ru). R' can be covalently or datively bound to the ruthenium metallic center, preferably R' is datively bound.
X is a ionic ligand chosen from the group consisting of halides (Cl⁻, Br⁻, I⁻), phenoxy (C₆H₆-O⁻), alkoxy (RO⁻), and hydroxyl (HO⁻). X can be also another anionic ligand suitable to covalently bond the metal center (M), as recited herewith: the ruthenium center.

In the context of the present invention, R can be a substituted or unsubstituted (linear) alkyl. R can also be a conjugated system: a linear alkylidene which may or may not have alternating C=C bonds with delocalized electrons. R can also be a non-conjugated linear hydrocarbon (alkyl or alkylidene). R can be an aryl, i.e. an aromatic cyclic hydrocarbon. R can be substituted and can comprise a functional group such as an ether (-O-), an amine or a phosphine that can establish a dative bond (i.e. not a conventional covalent bond but a dipole interaction between one free electron pair of the functional group such as an the electron pair on an oxygen atom) with the metal center. In this case, R is acting as a bidentate ligand towards the metallic center. In this case, n=0 and R' is absent, with R providing a ligand having one carbon atom bound by a double bond with the metallic center and a dative bond between a different atom and the metallic center.
R' can be a trisubstituted phosphine, such as a trialkylphosphine or a tricycloalkylphosphine or a triarylphosphine, wherein any of the alkyl groups (or all of the three alkyls) of the trialkylphosphine is an alkyl chosen from the group consisting of a methyl, an ethyl, a propyl, i-propyl, butyl, methyl-butyl, i-butyl, t-butyl, and wherein the tricycloalkylphosphine is a tricyclohexyl phosphine or a cyclohexyl dialkyl phosphine, or a dicyclohexyl alkyl phosphine. If R' is a substituted or unsubstituted pyridine, a substituted or unsubstituted pyrazine, the pyridine or pyrazine can be substituted by an alkyl, a halogen, or a hydroxyl.
R" can be a substituted imidazole derivative or a substituted dihydroimidazole derivative: the substitution occurs on one or both of the -N atoms, and is preferably an aromatic group such as a phenyl, a o-methyl phenyl, a m-methyl phenyl, a p-methyl phenyl, an o, o'-dimethyl phenyl, a m, m'-dimethyl phenyl, an o, m-dimethyl phenyl, an o, p-dimethyl phenyl, an m, p-dimethyl phenyl, an o,o',p-trimethyl phenyl, an m,m',p-trimethyl phenyl, or a o, m, p-trimethyl phenyl. The substituents on the aromatic ring (or one of the substituents) can alternatively be an isopropyl group with a similar substitution pattern as described above.

In the context of the present invention, the term 'substituted' represents a substitution chosen from the group consisting of a halogen atom (such as -F, -Cl, -Br, -I), a lower alkyl rest such as methyl, ethyl, n-propyl, i-propyl, n-butyl, i-butyl, t-butyl, a cycloalkyl such as cyclohexyl, an aryl, such as a phenyl, a hydroxyl (-OH), a oxy-alkyl (ether), such as a -O-methyl, -O-ethyl, -O-propyl, -O-(i-propyl), -O-butyl, -O-i-butyl, -O-t-butyl.

In the process according to the present invention, the ruthenium-based catalyst is advantageously selected from the group consisting of the molecules having formulae: 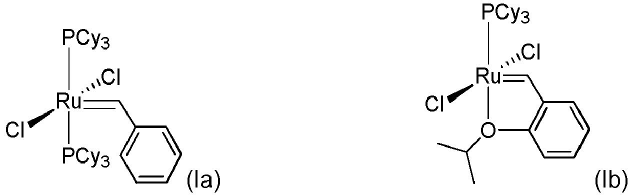 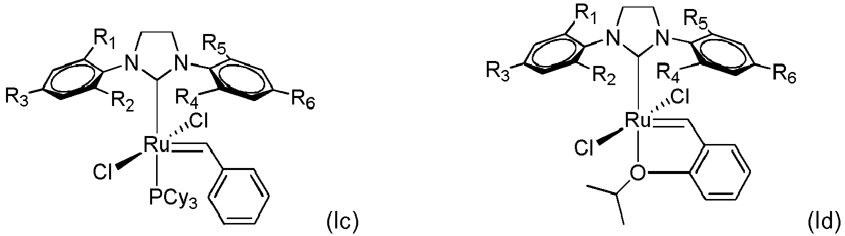 and 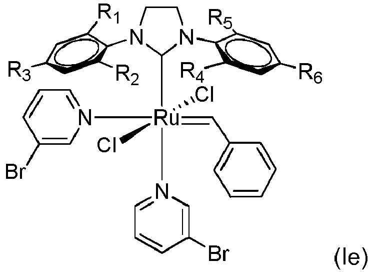 wherein Cy represents a cyclohexanyl substituent and R¹, R², R³, R⁴, R⁵ and R⁶ are substituents representing independently -H, an alkyl, a cycloalkyl, an aryl, a halogen, an -OH such as defined hereabove.

In the context of the present invention, the catalyst is not carrier-bound. In other words, the catalyst is not supported by or on an oxide of metal, such as TiO₂, SiO₂, ZrO₂, MgO or mixture thereof or a silicate of metal. The catalyst in the context of the present invention, is not supported on any activated surface, such as on activated carbon.

In the context of the present invention, the ligand comprises at least one donor atom chosen from the group consisting of a nitrogen atom and a phosphorus atom. The term 'donor atom' is to be understood as an atom which has at least one free electron pair which can interact and bond to the metallic center. In other words, the ligand comprises at least one functional group chosen from the group consisting of amine and phosphine. The ligand can comprise a primary, secondary or tertiary amine and/or the ligand can comprise a tertiary phosphine. Advantageously, the ligand comprises at least two functional groups selected from an amine and a phosphine. Accordingly, the ligand can comprise an amine and a phosphine group, or two amine groups, or two phosphine groups.

According to the present invention, one preferred ligand used to modify the metathesis catalyst and transform it into an ester hydrogenation catalyst has the formula (1):

₂HNCH₂CH₂P(R⁷)₃ (1)

wherein R⁷ is chosen from the group consisting of alkyl, cycloalkyl, aryl. When R⁷ is an alkyl, R⁷ is preferably a methyl, an ethyl, a propyl, i-propyl, butyl, methyl-butyl, i-butyl, t-butyl. When R⁷ is a cycloalkyl it is preferably a substituted or unsubstituted cycloalkyl. In the context of the present invention, a ligand is a molecule with electron donor atoms (having at least one free electron pair) which can bond to the metallic core via a non-conventional covalent bond (dative bond). The ligand can also be (or comprise a group having the formula): (R⁸)₂P(OR⁹) (phosphinite), (R⁸)P(OR⁹)₂ (phosphonite) or amino-phosphine (R⁸)P(NR⁹₂)₂ or (R⁸)₂P(NR⁹), wherein R³ and R⁹ can be identical or different and are chosen from the group consisting of alkyl, cycloalkyl, aryl. In the context of the present invention, the ligand does preferably not contain a -S- (sulfur) donor atom. Indeed, it has surprisingly been found in the context of the present invention, that ligands comprising at least one sulfur do not provide a suitable ligand to obtain a catalyst suitable for hydrogenation of an ester according to the process of the present invention. Advantageously, good results have been obtained when the ligand is present in an amount in the range from 1 to 2 molar equivalents relative to the molar equivalent of the catalyst.

According to the present invention, the base can be advantageously chosen from the group consisting of an alkoxide of an alkali metal such as Li⁺ salts, Na⁺ salts, K⁺ salts. Examples are advantageously, CH₃OLi, CH₃ONα, CH₃OK, CH₃CH₂OLi, CH₃CH₂ONa, CH₃CH₂OK, CH₃(CH₂)₂OLi, CH₃(CH₂)₂ONa, CH₃(CH₂)₂OK, (CH₃)₂CHOLi, (CH₃)₂CHONa, (CH₃)₂CHOK, (CH₃)₃CONa, (CH₃)₃COK. In the context of the present invention, a base is a molecule which can accept hydrogen cations (or protons), therefore having the property of deprotonating other compounds (Brønsted-Lowry definition). Advantageously, good results have been obtained when the base is present in an amount in the range from 10 to 20 molar equivalents relative to the molar equivalent of metal in the catalyst.

In the context of the present invention, step b) is a metathesis step which produces an ester product of the metathesis (a different ester than the starting product). The metathesis step can be a self-metathesis, when in step a) the aliphatic ester having at least one carbon-carbon double bond is the only compound provided. In this case a diester will be obtained in step b) and the hydrogenation product thereby obtained in step d) is a diol. Depending on the number of ester functions on the starting compound, it is possible to obtain triols, tetraols, pentaols, for example. The metathesis step can be a cross-metathesis, when in step a) the aliphatic ester having at least one carbon-carbon double bond is not the only compound provided (i.e. an olefinic compound is also provided). The product obtained in step b) is accordingly an ester product of the metathesis which results from the metathesis of an ester comprising at least one carbon-carbon double bond and of an (olefinic) compound comprising at least one carbon-carbon double bond which can react in a metathesis reaction. Therefore, the hydrogenation of the ester product of the metathesis allows obtaining an alcohol. Accordingly, the process of the present invention is a process for the manufacture of an aliphatic saturated alcohol (preferably α-ol, or α,ω-diol, or another diol having two primary alcohols functions).

In one embodiment of the present invention, an olefinic compound having at least one carbon-carbon double bond is provided in step a) together with the aliphatic ester having at least one carbon-carbon double bond, in this case, the metathesis step, step b) can be called a cross-metathesis step (leading to an alkenoate). The process of the present invention is in this case a process for the manufacture of an aliphatic saturated (primary) alcohol. The olefinic compound can be any substituted compound having the formula: H₂C=CHR*, wherein R* represents H, methyl, ethyl, propyl, i-propyl, butyl, methyl-butyl, i-butyl, t-butyl, a (linear or branched) alkyl comprising from six to 18 carbons, cyclohexyl, or phenyl.

In another embodiment of the present invention, an aliphatic ester having at least one carbon-carbon double bond is provided in step a) together with a second (different) aliphatic ester. In other words, in step a) at least one (such as one, two or more, three or more) aliphatic ester having at least one carbon-carbon double bond can be provided, such as two aliphatic esters having at least one carbon-carbon double bond. In this case, the metathesis reaction forms three products: self-metathesis of the first ester (ester A), self-metathesis of second ester (ester B), and cross-metathesis of the two esters (ester A with ester B). All of them will be reduced to alcohols. Accordingly, when two different aliphatic esters having at least one carbon-carbon double bond are provided in step a) the metathesis occurs between two (different) molecules of esters, thereby step b) is a cross-metathesis and a self-metathesis reaction. After step b) the product of the metathesis of the two (or more) esters is a mixture of alkene-dioates. The process of the present invention is in this case a process for the manufacture of an aliphatic saturated diol (wherein the alcohol functions are primary alcohols). The two esters herewith, also designated by alkyl alkenoates can have different alkyl rests or different alkene main chain. In other words, the alkyl alkenoates have the formula: R^{#}-COO-R^, wherein at least one of R^{#} and R^ are different. R^{#} is an alkenyl chain of any length according to the preferences recited above and R^ is an alkyl of any length, such as an alkyl chosen from the group consisting of methyl, ethyl, n-propyl, i-propyl, n-butyl, i-butyl and t-butyl. R^ can also be an aryl, such as phenyl. R^{#} and/or R^ may or may not be substituted. If R^{#} and/or R^ are substituted, the substitution can be chosen from the group consisting of a halogen atom (such as -F, -Cl, -Br, -I), a lower alkyl rest such as methyl, ethyl, n-propyl, i-propyl, n-butyl, i-butyl, t-butyl, a cycloalkyl such as cyclohexyl, an aryl, such as a phenyl, a hydroxyl (-OH), a oxy-alkyl (ether), such as a -O-methyl, -O-ethyl, -O-propyl, -O-(i-propyl), -O-butyl, -O-i-butyl, -O-t-butyl.

In yet another embodiment of the present invention, the aliphatic ester having at least one carbon-carbon double bond is the only product provided in step a) and therefore the metathesis occurs between two molecules of esters, thereby step b) is a self-metathesis reaction. After step b) the product of the metathesis is an alkene-dioate. The process of the present invention is in this case a process for the manufacture of an aliphatic saturated diol having two primary alcohol functions (such as a α,ω-diol).

According to the present invention, a step b1) can be carried out after step b) and before step c) wherein a (second) olefin is added to the first reaction mixture and a second metathesis reaction is carried out between ester product resulting from the metathesis reaction in step b) and the (second) olefin. Preferably, the (second) olefin is a cyclic olefin (cycloalkene) having at least 5 carbon atoms, more preferably at least 8 carbon atoms. The cyclic olefin is advantageously chosen from the group consisting of cyclopentene, cyclohexene, cycloheptene, cyclooctene, cyclononene, cyclodecene, cyclododecene, norbonene, norbonadiene. The cyclic olefin chosen herewith can be substituted or not. If the cyclic olefin is substituted, the substitution can be chosen from the group consisting of a halogen atom (such as -F, - Cl, -Br, -I), a lower alkyl rest such as methyl, ethyl, n-propyl, i-propyl, n-butyl, i-butyl, t-butyl, a cycloalkyl such as cyclohexyl, an aryl, such as a phenyl, a hydroxyl (-OH), a oxy-alkyl (ether), such as a -O-methyl, -O-ethyl, -O-propyl, -O-(i-propyl), -O-butyl, -O-i-butyl, -O-t-butyl. If the (second) olefin is a cycloalkene, step b1) is a ring-opening-metathesis-polymerisation (ROMP) step. ROMP is a type of olefin metathesis chain-growth polymerization that involves cyclic olefins (e.g. norbornene or cyclopentene). The addition of substituents to the olefin and the choice of solvent can alter the molecular weight of the polymer produced. The ROMP catalytic cycle requires a strained cyclic structure because the driving force of the reaction is relief of ring strain. After formation of the metal-carbene species, the carbene attacks the double bond in the ring structure forming a highly strained metallacyclobutane intermediate. The ring then opens giving the beginning of the polymer: a linear chain double bonded to the metal with a terminal double bond as well. The new carbene reacts with the double bond on the next monomer. This embodiment has advantageous applications to manufacture oligomers or polymers (comprising at least one alcohol function). One of the advantages of this embodiment of the present invention, is that the same catalyst can be used for step b) and for step b1). A further advantage is that this embodiment allows the formation of a molecule having at least 50 carbon atoms, preferably at least 90 carbon atoms, more preferably at least 100 carbon atoms, most preferably at least 120 carbon atoms (the ROMP reaction comprises the polymerization of the molecule obtained after the ring-opening and metathesis successive reactions). Step b1) can be repeated at least two times, such as 5 times, 10 times, 100 times or more with the same (second) olefin or a different (second) olefinic compound. Step b1) can be carried out with different amounts of equivalents of olefin in step b1), such as 1 or more equivalent, 2 or more equivalents, 3 or more equivalents, 4 or more equivalents, 5 or more equivalents, 10 or more equivalents, 20 or more equivalents., 50 or more equivalents (relative to the ester product resulting from the metathesis reaction of step b).

In the context of the present invention, any of the steps of the process can be carried out in a solvent. Any of the (or all) steps of the process according to the present invention can also be carried out without a solvent. In the context of the present invention, a solvent is an organic compound inert in the reaction and can be chosen from the group consisting of tetrahydrofuran (THF), methyl tetrahydrofuran (Me-THF), dioxane, dichloromethane (DCM), dimethylsulfoxide (DSMO), toluene and xylene.

According to one embodiment of the present invention, after step b), the ester product of the metathesis is isolated before carrying out the hydrogenation in step d), for example by a distillation process, by a filtration process or a combination thereof.

According to one embodiment of the present invention, the process is carried out without isolating the product of the metathesis of the ester, before carrying out the homogeneous hydrogenation. Accordingly, the metathesis of the unsaturated ester is directly followed by the homogeneous hydrogenation. The advantage of such embodiment is that the process can be carried out in one pot, therefore providing a 'one-pot process', having technical advantages such as preventing removal of the catalyst between step b) and step d) and cost advantages since the catalyst used in step b) is further used in the process according to the present invention. The same (precious) metal contained in the catalyst is used for the two different reactions of step b) and d). A further advantage of the present invention is that no removal of solvent is necessarily carried out in the process according to the present invention. A solvent may be present in any of (or all) steps according to the present invention, however the presence of a solvent is not necessary.

In the context of the present invention, all the definitions and preferences recited for the process for the manufacture of the saturated alcohol hereabove are applicable to all aspects of the present invention.

One other aspect of the present invention is a ruthenium-based catalyst suitable for the homogeneous hydrogenation of esters obtainable by the process comprising step of:
i) Dissolving the catalyst as defined in any of formulae (I), (Ia), (Ib), (Ic), (Id) and (Ie) in a solvent;
ii) Dissolving a ligand comprising at least one amino group and at least one phosphine group into a solvent;
iii) Mixing the solutions obtained in i) and ii) and adding a base thereby obtaining a ruthenium-based catalyst in solution suitable for the homogeneous hydrogenation of esters and optionally,
iv) Isolating the ruthenium-based catalyst by a separation process chosen from the group consisting of crystallization, filtration, centrifugation, distillation or any combination thereof (in order to remove eventual unreacted ligand, and/or base and/or solvent(s)).

In the context of the present invention, a similar catalyst as described herewith may be suitable for the homogeneous hydrogenation of esters according to the process of the present invention wherein the catalyst is an osmium-carbene based catalyst.

The solvent present in the process for the manufacture of the above-mentioned catalyst or in the process for the manufacture of a saturated alcohol according to the present invention is an inert component which can be chosen from the group consisting of tetrahydrofuran (THF), methyl tetrahydrofuran (Me THF), dioxane, dichloromethane (DCM), dimethylsulfoxide (DSMO), toluene and xylene. The solvent in steps i) and ii) may be identical or different. Steps i) and ii) may occur in one pot or separately. If steps i) and ii) occur in one pot, the mixing in step iii) is carried out under agitation. If steps i) and ii) are carried out separately, the mixing in step iii) is to be understood as adding the solution obtained after step i) to the solution obtained after step ii) or adding the solution obtained after step ii) to the solution obtained after step i). Steps i) and/or ii) and/or iii) may occur under agitation using any agitation, such as magnetic, mechanic or by ultrasound. A steps i) and ii) can occur at a stirring speed of at least 50 rpm and step iii) may occur a stirring speed of at least 300 rpm under agitation. Steps i) and/or ii) and/or iii) may occur at room temperature (between 10°C and or 40°C) or under some heating. Step iii) is preferentially performed under inert atmosphere: under an oxygen-free atmosphere such as nitrogen or argon atmosphere. Steps ii) and iii) may be designated as the process steps c) in the process for the manufacture of a saturated alcohol from an unsaturated aliphatic ester according to the present invention.

The advantages of the above catalyst is that it can be used to hydrogenate the ester functional group into an alcohol in a homogeneous hydrogenation reaction and under mild conditions, such as a hydrogen pressure below 100 bar and at a temperature below 250°C, preferably at most at 200°C, more preferably at most at 180°C , most preferably at most at 170°C. A particular advantage of the present invention, is that the temperature of the hydrogenation step can be carried out at low temperatures. The minimum temperature for the hydrogenation is at least 40°C , preferably at least 50°C, more preferably at least 60°C.

The conversion yield of the hydrogenation according to the present invention, using the catalyst according to the present invention is at least 85 % and can reach conversion yield as high as 99%.

Yet one other aspect of the present invention is a process for the homogeneous hydrogenation of an ester into an alcohol comprising contacting an ester with hydrogen in the presence of a ruthenium-based catalyst as defined hereabove.

According to an embodiment of the present invention, the hydrogen pressure of the hydrogenation reaction can be below 100 bar and the temperature of the hydrogenation reaction can be below 250°C, advantageously even below 170°C, more advantageously even below 150°C, most advantageously below 100°C. This provides the advantage of a process occurring in mild conditions compared to known hydrogenation methods.

Advantageously, the hydrogenation conditions in the process according to the present invention are a low pressure. The low pressure is in the range from 10 to 70 bar, preferably 10 to 50 bar. Advantageously, the temperature is in the range from 40°C to 100°C, more advantageously from 55°C to 85°C, most advantageously from 65°C and 75°C. A pressure in the range from 40 to 70 bar with a temperature between 65°C and 75°C provides an outstanding reaction yield. The above pressure and/or temperature ranges thereby providing a process particularly suitable in large industrial plants, where volume, pressure and temperature are critical.

In the context of the present invention, all the definitions and preferences recited for the catalyst for the manufacture of the saturated alcohol (by hydrogenation of an ester) and the process for the hydrogenation of an ester as described hereabove are applicable to all aspects of the present invention.

### EXAMPLES

### Example 1: self-metathesis and hydrogenation to produce an α,ω-diol

In a N2 glove-box, catalyst (Ia) was placed into a 5 mL vial. 4-methyl pentenoate was added and the reaction mixture was stirred at 30°C for 3h. Ethylene gas was produced. The ligand NH₂CH₂CH₂P(C₆H₅)₂ was dissolved in 1 ml of dry THF. The metathesis reaction mixture was added to the ligand solution. After stirring, the reaction mixture was added to a vial containing t-BuOK. The reaction was placed in the hydrogenation reactor under 50 bar of hydrogen at 70°C for 16 h.

The conversion yield was determined based on the initial 4-methyl pentenoate and converted into the desired product: 1,8-octane diol. The yield of the metathesis reaction was 88%, the yield of the hydrogenation reaction was 95%, therefore the overall yield was 84%.

### Example 2: cross-metathesis and hydrogenation to produce an alcohol

In a N₂ glove-box, catalyst (Ib) was placed into a 5 mL vial. A mixture of styrene and methyl acrylate was added and the reaction mixture was stirred at 30°C for 3h. Ethylene gas was produced.

The ligand NH₂CH₂CH₂P(C₆H₅)₂ was dissolved in 1 ml of dry THF. The metathesis reaction mixture was added to the ligand solution. After stirring, the reaction was added to a vial containing t-BuOK. The reaction was placed in the hydrogenation reactor under 50 bar of hydrogen at 70°C for 16 h.

The conversion yield was determined based on the initial styrene and converted into the desired product: 3-phenyl propanol. The yield of the metathesis reaction was 66%, the yield of the hydrogenation reaction was 89%, therefore the overall yield was 59%.

### Comparative Experiments

α,ω-diols can be prepared by metathesis of fatty acid ester/hydrogenation using 2 different catalysts: the metathesis catalyst used above and a traditional ester hydrogenation catalyst such as an heterogeneous catalyst operating at very high pressure (>150 bar) and temperature (>250°C). Such a process absolutely requires isolation of the product after the first metathesis step, two different catalysts and a dedicated high pressure reactor. Such a process is therefore not as versatile and cost-effective as the process according to the present invention.

In processes wherein the metathesis catalyst was not modified by the addition of ligand and used as such in the hydrogenation reaction, the ester group remained unchanged during the hydrogenation step indicating that the unmodified metathesis catalyst is not active towards ester hydrogenation. These processes did not allow to produce alcohols from esters.

## Claims

1. Process for the manufacture of a saturated primary alcohol from an unsaturated aliphatic ester comprising the steps of:
a) Providing an aliphatic ester having at least one carbon-carbon double bond;
b) Carrying out a metathesis of said ester in the presence of a ruthenium carbene-based catalyst thereby obtaining a first reaction mixture;
c) Adding a ligand and a base to the first reaction mixture, wherein the ligand comprises at least one donor atom chosen from the group consisting of a nitrogen atom and a phosphorus atom thereby obtaining a second reaction mixture comprising an ester product resulting from the metathesis reaction ;
d) Carrying out a homogeneous hydrogenation of the ester-product resulting from the metathesis, thereby obtaining a saturated primary alcohol.

2. Process according to claim 1, wherein a second compound having at least one carbon-carbon double bond is provided in step a) together with the aliphatic ester having at least one carbon-carbon double bond.

3. Process according to claim 1 or 2, wherein a step b1) is carried out after step b) and before step c) comprising adding an olefin to the first reaction mixture and carrying out a second metathesis between the ester product resulting from the metathesis reaction in step b) and the olefin.

4. Process according to claim 3, wherein the olefin is a cyclic olefin and step b1) is a ring-opening-metathesis polymerisation.

5. Process according to any one of claims 1 to 4, wherein the ester provided in step a) is an ester of an unsaturated fatty acid.

6. Process according to any one of claim 1 to 5, wherein the catalyst has the formula (I): 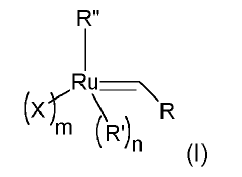 wherein
m and n are independently numerals between 0 and 2,
X is a ionic ligand chosen from the group consisting of halides, phenoxy, alkoxy and hydroxyl,
R is chosen from the group consisting of an unsubstituted linear alkyl, a substituted linear alkyl, an unsubstituted aryl and a substituted aryl,
R' is chosen from the group consisting of an unsubstituted trialkylphosphine, a substituted trialkylphosphine, an unsubstituted tricycloalkylphosphine, a substituted tricycloalkylphosphine, an unsubstituted triarylphosphine, a substituted triarylphosphine, an unsubstituted pyridine, a substituted pyridine, an unsubstituted pyrazine and a substituted pyrazine;
and
R" is chosen from the group consisting of a substituted imidazole-2-ylidene or a substituted dihydroimidazole-2-ylidene, an unsubstituted trialkylphosphine, a substituted trialkylphosphine, an unsubstituted tricycloalkylphosphine, a substituted tricycloalkylphosphine, an unsubstituted triarylphosphine, a substituted triarylphosphine.

7. Process according to any one of claims 1 to 6, wherein the ligand has the formula (1): NH₂CH₂CH₂P(R⁷)₃, wherein R⁷ is chosen from the group consisting of alkyl, cycloalkyl, aryl.

8. Process according to any one of claims 1 to 7, wherein the base is an alkoxide of an alkali metal.

9. Process according to any one of claims 1 to 8, wherein after step b), the ester product of the metathesis is isolated before carrying out the hydrogenation in step d).

10. Process according to any one of claims 1 to 8, wherein the process is carried out without isolating the ester product of the metathesis, before carrying out the homogeneous hydrogenation.

11. Ruthenium-based catalyst suitable for homogeneous hydrogenation of esters obtainable by the process comprising step of:
i) Dissolving the catalyst as defined in formula (I) in a solvent;
ii) Dissolving a ligand comprising at least one donor atom chosen from the group consisting of a nitrogen atom and a phosphorus atom in a solvent;
iii) Mixing the solutions obtained in i) and ii) and adding a base thereby obtaining a ruthenium-based catalyst in solution suitable for the homogeneous hydrogenation of esters.

12. Process for the homogeneous hydrogenation of an ester into an alcohol comprising contacting an ester with hydrogen in the presence of a ruthenium-based catalyst as defined in claim 10.

13. Process according to claim 12, carried out under a hydrogen pressure below 100 bar and at a temperature below 250°C.

14. Process according to claim 13, wherein the hydrogen pressure is in the range from 10 to 70 bar and the temperature is in the range from 55°C to 85°C.
